# EUROPEAN PATENT SPECIFICATION

(11) **EP 0 484 378 B1**
(45) Date of publication and mention of the grant of the patent: **14.09.1994**
(21) Application number: 90911059.5
(22) Date of filing: 19.07.1990
(51) Int. Cl.: C07C 311/08, C07D 295/135, C07D 213/74, A61K 31/18, A61K 31/395, A61K 31/55, A61K 31/495

(54) **ANTIARRHYTHMIC TERTIARY AMINE-ALKENYL-PHENYL-ALKANESULFONAMIDES**
TERTIÄRAMIN-ALKENYLPHENYLALKANSULFONAMIDE MIT ANTIARRHYTHMISCHER WIRKUNG
AMINE-ALCENYLE-PHENYLE-ALCANESULFONAMIDES TERTIAIRES ANTIARYTHMIQUES

(30) Priority: 25.07.1989 US 385335
(43) Date of publication of application: 13.05.1992
(73) Proprietor: THE UPJOHN COMPANY, Kalamazoo, Michigan 49001 (US)
(72) Inventor: HESTER, Jackson, Boling, Jr., Galesburg, MI 49053 (US); PERRICONE, Salvatore, Charles, Otsego, MI 49078 (US); GIBSON, John, Kenneth, Kalamazoo, MI 49001 (US)
(74) Representative: Perry, Robert Edward
(86) International application number: US9003960
(87) International publication number: WO9101299

(56) References cited:
- EP-A- 0 164 865
- EP-A- 0 304 888
- US-A- 3 758 692

## Description

The present invention is directed toward compounds having an alkenyl linkage between a tertiary amine group and an alkanesulfonamide substituted phenyl. These novel alkanesulfonamides prolong the effective refractory period of the myocardium and are useful for treating cardiac arrhythmias.

Antiarrhythmic drugs act upon the electrophysiological properties of the myocardium and conductive tissues. Typically the rhythmic contractions of the heart are dependent upon the ability of the myocardium and conductive tissues to respond to electrical impulses. When the conductivity of the heart's muscle and conductive tissue is altered by an occlusion of an artery or disease, a life threatening cardiovascular deterioration is likely. It is therefore desirable to treat the electrophysiological properties of the myocardium and conductive tissue to restore rhythmic contractions.

One means for restoring rhythmic contraction is with an antiarrhythmic agent that selectively prolongs the action potential duration and concomitantly increases the refractory period of heart cells without significant effect on cardiac conduction. Such drugs are classified as Class III antiarrhythmic agents. Class III antiarrhythmics which have good bioavailability and which do not affect other circulatory parameters such as blood pressure and heart rate are continually being sought. The subject compounds are Class III antiarrhythmics which are suitable for the treatment of mammals suffering from antiarrhythmic disorders or disease.

### INFORMATION DISCLOSURE STATEMENT

The subject compounds are generally related to those compounds described in European Patent No. 0164865. These compounds can be used as intermediates in the preparation of the subject compounds.

European Patent Application EP 0134424 discloses quaternary ammonium salts of compounds which are isomers of the subject alkanesulfonamides.

T. K. Morgan, Jr. et al., J. Med Chem., 29, 1398 (1986) reports tertiary amine alkanesulfonamides compounds.

U.S. Patents 3,341,584 and 3,478,149 disclose sulfonamide compounds some of which can be used as intermediates for the preparation of the subject compounds.

Other U.S. Patents having examples of sulfonamide containing compounds and antiarrhythmic activity are DeMarinis et al. 4,507,320, Molloy et al. 4,569,801 and 4,596,827, and Gould et al. 3,574,741.

### SUMMARY OF THE INVENTION

In one aspect the subject invention is directed toward a compound of Formula I and pharmacologically acceptable salts thereof. Formula I is defined where n is 1 to 3; R is a C₁₋₄ alkyl; R₁ is hydrogen or C₁₋₄ alkyl; R₂ is a C₁₋₁₀ alkyl; R₃ is a C₁₋₁₀ alkyl (which can be substituted with from one to eight fluorine atoms, or one to three hydroxy, one to three C₁₋₅acyloxy or one to three C₁₋₄alkoxy substituents), a C₃₋₁₀ cycloalkyl, a C₃₋₁₀ alkenyl, a C₁₋₇ alkyl substituted with an aryl, heteroaryl or C₃₋₇ cycloalkyl, and where the sum of carbons in R₂ and R₃ is greater than five or where R₂ and R₃ with the nitrogen atom form a saturated heterocyclic group having one nitrogen and from 4-8 carbon atoms or a 4-substituted piperazine group in which the 4-substituent can be C₁₋₁₀ alkyl, aryl, benzyl, or heteroaryl; and X is hydrogen, hydroxy, C₁₋₄ alkoxy, C₁₋₄ alkyl, carbon trifluoride or a halogen. Preferred compounds of Formula I are where X and R₁ are hydrogen. Also preferred are compounds where only one occurrence of R₁, is an alkyl. An example of a compound of Formula I is (E)-N-(4-(4-(ethyl-heptylamino)-1-butenyl)phenyl)methanesulfonamide, (E)-2-butenedioate (2:1 salt).

In another aspect the subject invention is directed toward a method for treating cardiac arrhythmia in mammals comprising the administration of a therapeutically effective amount of a compound of Formula I including pharmacologically acceptable salts thereof. An effective amount is from about 0.01 to about 300 mg. Preferably, the compound is administered in a unit dosage form for oral, sublingual, transdermal or parenteral administration.

The Formula I compounds are generally prepared into pharmacological preparations or compositions for therapeutic administration to patients suffering from cardiac arrythmia. The compounds are classified as Class III antiarrhythmic compounds which are agents that selectively prolong the action potential duration and concomitantly increase the refractory period of heart cells without significant effects on cardiac conduction.

### DETAILED DESCRIPTION OF THE SUBJECT INVENTION

Alkanesulfonanilides which prolong the effective refractory period of the myocardium and are useful for treating cardiac arrhythmias in mammals are disclosed. The compounds of the present invention are represented by the structural Formula I, shown on the formula sheet below, and its pharmaceutically acceptable salts. Formula I is defined where n is 1-3; R is a C₁₋₄ alkyl; R₁ is hydrogen or C₁₋₄ alkyl, preferably only one occurrence of R₁ is an alkyl; R₂ is a C₁₋₁₀ alkyl; R₃ is a C₁₋₁₀ alkyl (which can be substituted with from one to eight fluorine atoms, one to three hydroxy, one to three C₁₋₅ acyloxy or one to three C₁₋₄ alkoxy substituents), C₁₋₇ alkyl substituted with an aryl, heteroaryl or C₃₋₇ cycloalkyl; C₃₋₁₀ cycloalkyl; C₃₋₁₀ alkenyl; and where the sum of carbons in R₂ and R₃ is greater than five; and X is hydrogen, hydroxy, C₁₋₄ alkoxy, C₁₋₄ alkyl, a halogen (fluorine, chlorine, bromine), or carbon trifluoride.

R₂ and R₃ taken together with the shared nitrogen can form a saturated heterocyclic group having one nitrogen and from 4-8 carbon atoms or a 4-substituted piperazine group in which the 4-substituent can be C₁₋₁₀ alkyl, aryl, benzyl, or heteroaryl. The aryl and heteroaryl substituents, defined below, are bond directly to the piperazine through a ring carbon.

An "alkyl" is a straight or branched carbon chain containing the number of carbon atoms designated such as C₁₋₄, C₁₋₅, C₁₋₁₀, etc. A "substituted" alkyl is a straight or branched carbon chain having a hydrogen atom replaced by another chemical group such as an aryl, heteroaryl or cycloalkyl. An "alkenyl" is a straight or branded carbon chain having three to ten carbon atoms and containing at least one degree of unsaturation. Halogen refers to the group of chlorine, fluorine, bromine or iodine. Halogen substituted means that one or more of the hydrogen atoms on a carbon are replaced with a halogen atom as demonstrated in Formula Ib where Z is either F or H.

An "aryl" is a phenyl or a substituted phenyl ring having one or two substituents such as a C₁₋₄ alkyl, C₁₋₄ alkoxy, hydroxy, C₁₋₄ alkanesulfonamide, or carboxyl including amides and esters thereof. The aryl group is either linked directly to the alkyl group or through an oxygen, nitrogen or sulfur atom.

"Heteroaryl" is defined as a five- or six-membered aromatic heterocycle which can contain one oxygen or sulfur atom and/or one to three nitrogen atoms, linked to the alkyl by a bond to a ring carbon or secondary nitrogen or through an exocyclic nitrogen atom, optionally substituted by one or two substituents selected from amino, hydroxy, C₁₋₄ alkoxy, C₁₋₄ alkyl or halogen;

An "alkoxy" is an alcohol or phenol in which the hydrogen attached to the oxygen is replaced with a straight or branched carbon chain.

A "cycloalkyl" is a cyclic ring structure formed from three to ten carbon atoms. The cyclic structure may also contain an alkyl substitution wherein the total carbons are calculated to include this substitution.

"Acyloxy" is an ester of a alcohol with a carboxylic acid having from one to five carbon atoms.

The RSO₂NH group is attached to the benzene ring at the positions meta or para to the side chain.

"Pharmacologically acceptable salts" are acid addition salts which can be prepared by any of the art recognized means. Typical, acid addition salts include hydrochloride, hydrobromide, hydroiodide, sulfate, phosphate, acetate, propionate, lactate, maleate, malate, succinate, tartrate, cyclohexanesulfamates, methanesulfonates, ethanesulfonates, benzenesulfonates, toluenesulfonates, fumarates and other pharmaceutically acceptable counter ions for amines.

The Formula I compounds are used for the treatment of arrhythmia wherever a Class III antiarrhythmic drug is indicated. The compounds and compositions of Formula I are administered in a therapeutic effective amount which is an amount sufficient to control arrhythmia in the host being treated such as mammals which includes humans. Typically, the Formula I antiarrhythmic agents are used in unit dosages of from 0.01 to 300 mg in oral or injectable preparations. Preferably, the Formula I compounds are used in unit dosages of 0.001 to 10 mg/kg for administration by routes either oral, sublingual, transdermal, or parenteral such as by subcutaneous, intramuscular, or intravenous injection.

The particular dose of compound administered according to this invention will of course be determined by the particular circumstances surrounding the case, including the compound administered, the route of administration, the particular arrhythmia being treated, and similar considerations.

The Formula I compounds can be formulated into typical pharmaceutical preparations for either oral or parenteral administration. For example, the Formula I compound can be formulated into a composition by admixing with any of a number of suitable pharmaceutical diluents and carriers such as lactose, sucrose, starch powder, cellulose, calcium sulfate, sodium benzoate and the like. Such formulations can be compressed into tablets or can be encapsulated into gelation capsules for convenient oral administration.

A gelatin capsule suited to oral administration may contain, for example, a Formula I compound in the amount of about 0.1 to about 100 mg. Such formulation can be administered orally as often as needed depending upon the particular condition and patient being treated.

For parenteral administration a Formula I compound can be formulated for intramuscular or intravenous administration. In the case of treatment of a patient suffering from a severe cardiac arrhythmia, it may be desirable to administer the Formula I compound by intravenous infusion in order to effect a speedy conversion to a normal cardiac rhythm. Such normal condition can then be maintained by oral administration.

The compositions of the present invention may also include sustained release oral dosage forms and controlled release dosage forms by which the effect of the dosage is through the skin. Such compositions are those known to an ordinary skilled artisan or can be ascertained by ordinary experimentation from known compositions such as creams, gels, pastes or liquids. Typical transdermal compounds are polyethylene glycol, triacetin, propylcarbonate, ethanol and isopropyl myristate.

The Formula I compounds can be combined with other antiarrhythmic agents having the same or different mechanisms of action. For example, combinations may include, Class I antiarrhythmic agents, such as quinidine, tocainide, lidocaine or the like; Class II antiarrhythmic agents, such as, propranolol, sotalol, atenolol or the like; Class III antiarrhythmic agents such as clofilium, sotalol, amiodarone and meobentine; and Class IV antiarrhythmic agents such as verapamil or diltiazem.

The Formula I compounds are prepared by dehydrating an appropriate benzylic alcohol. Examples of suitable starting materials are described in European Patents 0 164 865 and 0 233 051, U.S. Patents 3,341,584, 3,478,149 all herein incorporated by reference. The dehydration procedure can be performed with trifluoroacetic acid in solvents such as chloroform or methylene chloride at temperatures of 0-40°C. Olefins with an (E) configuration (trans) are generally the major products which are also the preferred products. Other intermediates useful in the preparation of the subject compounds are those alcohols not disclosed in the cited patents, where the corresponding R₃ group is a fluorinated alkyl, an alkenyl or an alkyl substituted by hydroxy, acyloxy or alkoxy or where R₃ is a C₁₋₇ alkyl substituted with an aryl, heteroaryl or C₃₋₇ cycloalkyl (Formula I'). Therefore these intermediates and their enantiomers are part of the subject invention.

The Formula I compounds were evaluated for electrophysiological activity in an isolated, perfused rabbit cardiac tissue system. The method used was as follows:
New Zealand White rabbits of either sex (1.5-2.0 kg) were anesthetized and their hearts removed. The heart was immersed in ice cold perfusate while the right atria (RA), papillary muscles (PAP), and right ventricular muscle strips (RV) were isolated. The perfusate was continuously oxygenated with 95% oxygen and 5% carbon dioxide and contained the following in mM concentrations: NaCl 118.0; KCl 5.4; NaHCO₃ 25.0; MgCl₂ 1.2; KH₂PO₄ 1.0; CaCl₂ 2.4; glucose 110.0 and pyruvic acid 2.0. During hypoxic conditions the perfusate was exposed to a mixture of 83% nitrogen, 10% carbon dioxide and 7% oxygen. The pH during normoxia was approximately 7.4 and dropped to approximately 7.2 during hypoxic conditions.

The tissues were individually mounted on a plexiglass holder containing platinum stimulating electrodes and suspended in a 100 ml bath maintained at 30°C by a circulating heat pump. All tissues were attached by silk suture to a force-displacement transducer and a tissue-dependent preload of 500-1000 mg was applied. RA were allowed to contract spontaneously. RV and PAP were stimulated at 2X threshold with 4 msec rectangular pulses at a frequency of 1 and 3 Hz. (Effective refractory period measurements are ERP1 and ERP3, conduction time measurements are CT1 and CT3). Between measurements those tissues were stimulated at a resting pace of 2 Hz. Each tissue served as its own baseline control and was allowed an equilibration period of two hours prior to experiments. During this period the perfusate was changed every 10-15 minutes.

Working solutions of the drugs were prepared by dissolving the drugs in distilled water and one drop of NaOH/ml to aid in dissolution (pH 9.4).

Measurements were made on each set of tissues after exposure to 10⁻⁷, 10⁻⁶ or 10⁻⁵M drug for 15 minutes; and 10⁻⁵M drug under hypoxic conditions for 15 minutes.

Automaticity (RATE), force of contraction (FOC) and threshold were measured directly on a polygraph. The ERP of cardiac tissues by definition is the longest coupling interval between the basic drive (S1) and the premature impulse (S2) that fails to propagate through the tissue. The S2 stimulus was introduced after every eighth S1 which allowed time for stabilization of refractoriness. Refractory period measurements were made via a digital timing circuit. The limit of resolution for these refractory period measurements was approximately 6 msec. Conduction time measurements (CT) were recorded directly in msec by gently placing a teflon-coated silver bipolar electrode against the endocardial surface of the RV strip with the resulting electrocardiogram displayed on an oscilloscope. An increase in CT is equivalent to a decrease in conduction velocity.

A Formula I compound tested was (E)-N-(4-(4-(Ethylheptylamino)-1-butenyl)phenyl)methanesulfonamide, (E)-2-butenedioate (2:1 salt) (see, Example 1) a dehydration product of the side chain hydroxyl moiety of N(4-(4-(ethylheptylamino)-1-hydroxybutyl)phenyl)methanesulfonamide, (E)-2-butenedioate (2:1 salt), described in EP 0164865. This compound caused substantial increases in both ERP1 and ERP3, increased the ERP during hypoxia and lengthened CT at 3Hz. An unusual aspect of this observation is the fact that during hypoxia only one of six papillary muscles was able to contract strongly enough to produce a readable signal. This particular system uses a contractile measurement to determine the refractoriness of the tissue. On occasion, one or perhaps two tissues in a group this size are not able to withstand hypoxia, but in this test 83% were unable to respond. For the one tissue that was able to contract, the ERP3 lengthened considerably. This suggests a very selective depression of cardiac activity during hypoxia with this compound.

**TABLE 1**

| Example 1 | FOC | ERP1 | ERP3 | RATE |
|---|---|---|---|---|
| | Mean | Mean | Mean | Mean |
| Baseline | 215 | 192 | 158 | 110 |
| 10⁻⁷M | 209 | 195 | 163 | 110 |
| 10⁻⁶M | 221 | 200 | 168 | 103 |
| 10⁻⁵M | 206 | 219* | 182* | 90 |
| Hypoxia | 25 | 207 | 197 | 57 |

Data are reported as raw mean values. An asterisk (*) denotes significance at p<0.05 vs control based on percent values. FOC = force of contraction in mg at 2.0 Hz. ERP1 = effective refractory period in milliseconds at 1.0 H_{z}. ERP3 = effective refractory period milliseconds at 3.0 H_{z}. RATE = spontaneous right atrial rate in beats per minute.

**Table 2**

| Conduction Time Results | | |
|---|---|---|
| Example 1 | 1Hz | 3Hz |
| 10⁻⁷M | -7 | 3 |
| 10⁻⁶M | -3 | 7 |
| 10⁻⁵M | 11 | 21* |
| Hypoxia | 48 | 79 |

Data are reported as percent baseline mean values. An asterisk (*) denotes significance at p < 0.05 vs. control.

### Example 1 (E)-N-(4(4-(Ethylheptylamino)-1-butenyl)phenyl)methanesulfonamide, (E)-2-butenedioate (2:1 salt) (Formula Ia)

To a mixture of 1.75 ml of trifluoroacetic acid and 1.75 ml of CH₂Cl₂ at room temperature, under nitrogen, was added 0.63 g of N-(4-(4-(ethylheptylamino)-1-hydroxybutyl)phenyl)methanesulfonamide. The mixture was stirred for 24 hours at room temperature. The volatiles were allowed to evaporate under a stream of nitrogen and the residue partitioned between EtOAc and saturated NaHCO₃. The organic extracts were pooled and washed with brine. After drying (MgSO₄) the organic solution was concentrated in vacuo. The residue was flash chromatographed over 200 ml of silica gel; elution with 15% MeOH/CHCl₃ (8 ml fractions were taken). Fractions 26-42 were pooled and concentrated to give 0.36 g of clean product. The ¹H NMR (300 MHz, CDCl₃) had: δ 6.40 (d,l,J = 16 Hz, ArCH = CH), 6.04 (p, l, ArCH = CH). This material was combined with product isolated from two previous runs and partitioned between EtOAc and 8% aqueous NaHCO₃ to give 0.96 g (2.62 mmol) of the free base which was combined with 0.152 g (1.31 mmol) of fumaric acid in ethanol. The mixture was concentrated to a small volume and treated with Et₂O to the cloud point; cooling produced crystallization. Recrystallization from EtOH/Et₂O gave 0.75 g of the hemifumarate, mp 112-3°. The NMR, mass spectrum and IR were consistent with the proposed structure. Anal. calcd for C₂₀H₃₄N₂O₂S0.5 C₄H₄O₄: C, 62.23; H, 8.55; N, 6.60; S, 7.55. Found: C, 62.11; H, 8.72, N, 6.52; S, 7.41.

### Example 2 (E)-N-(4-(4-(Hexahydro-1H-azepin-1-yl)-1-butenyl)phenyl)methanesulfonamide

(E)-N-(4-(4-(Hexahydro-1H-azepin-1-yl)-1-butenyl)phenyl)methanesulfonamide (2.0 g, 5.87 mmol) was dissolved in 7 ml of CH₂Cl₂, the mixture was cooled in an ice bath and treated dropwise with 7 ml of trifluoroacetic acid over 20 minutes. This mixture was stirred at room temperature for 48 hours and the volatiles were removed under a stream of N₂. The residue was diluted with EtOAc and washed twice with cold dilute NaHCO₃ and once water. The aqueous washes were combined and extracted with EtOAc. The organic extracts were combined, washed with brine, dried (MgSO₄) and concentrated to give 1.92 g of crude material. Chromatography over silica gel with 0.8% NH₄OH, 8% MeOH/CH₂Cl₂ gave 0.73 g (38.6%) of product. The analytical sample was crystallized from Et₂O/pentane, and had m.p. 73-4°C. The IR, NMR and mass spectrum supported with the proposed structure. Anal calcd. for C₁₇H₂₆N₂O₂S: C, 63.32; H, 8.12; N, 8.69; S, 9.95. Found: C, 62.94; H, 8.00; N, 8.44; S, 9.95.

### Example 3 (E)-N-(4-(4-(Dibutylamino)-1-butenyl)phenyl)methanesulfonamide, (E)-2-Butenedioate (2:1 salt)

A solution of N-(4-(4-(dibutylamino)-1-hydroxybutyl)phenyl)methanesulfonamide, (2.08 g, 5.48 mmol) in 8 ml of CH₂Cl₂ was cooled in an ice bath and treated dropwise with 8 ml of CF₃COOH over 10 minutes. This mixture was stirred at room temperature for 48 hours. The volatiles were removed under a stream of nitrogen; the residue was diluted with EtOAc and washed twice with cold, dilute NaHCO₃. The pooled aqueous wash was extracted with additional EtOAc. The pooled organic extract was washed with brine, dried (MgSO₄) and concentrated to give the crude product. Chromatography over silica gel with 0.8% NH₄OH' 8% MeOH/CH₂Cl₂ gave 1.11 g (49.3%) of product. The hemifumarate was prepared and crystallized from acetone to give 1.07 g, m.p. 131-2°C (softening at 124°). The IR, NMR and mass spectrum supported the proposed structure. Anal. calcd for C₁₉H₃₂N₂O₂S 0.5C₄H₄O₄: C, 61.43; H, 8.35; N, 6.82; S, 7.81; Found: C, 61.44; H, 8.71; N, 6.53; S, 7.48.

### Example 4

In the process as described in Example 2 the starting alcohols listed in Table 3 are treated with trifluoroacetic acid to give the corresponding products listed in Table 4 which are compounds of Formula I. The preparation of the starting alcohols (1-7) can be found in European Patent 0 164 865 and starting alcohol (8) can be found in European Patent 0 233 051.

**Table 3**

| | |
|---|---|
| 1) | N-(4-(4-(Dipropylamino)-1-hydroxybutyl)phenyl)isopropanesulfonamide |
| 2) | N-(4-(3-(Ethylheptylamino)-1-hydroxypropyl)phenyl)methanesulfonamide |
| 3) | N-(4-(3-(Hexamethyleneimino)-1-hydroxypropyl)phenyl)methanesulfonamide |
| 4) | N-(4-(3-(Dibutylamino)-1-hydroxypropyl)phenyl)methanesulfonamide |
| 5) | N-(4-(4-(Heptamethyleneimino)-1-hydroxybutyl)phenyl)methanesulfonamide |
| 6) | N-(3-(4-(Ethylheptylamino)-1-hydroxybutyl)phenyl)methanesulfonamide |
| 7) | N-(4-(4-Decylethylamino)-1-hydroxybutyl)phenyl)methanesulfonamide |
| 8) | N-(4-(1-hydroxy-3-(4-(4-pyridinyl)-1-piperazinyl)propyl)phenyl)methanesulfonamide |
| 9) | N-(4-(4-(hexamethyleneimino)-1-hydroxypentyl)phenyl)methane sulfonamide |
| 10) | N-(4-(4-(dibutylamino)-1-hydroxypentyl)phenyl)methanesulfonamide |

**Table 4**

| | |
|---|---|
| 1) | (E)-N-(4-(4-(Dipropylamino)1-butenyl)phenyl)isopropanesulfonamide |
| 2) | (E)-N-(4-(3-(Ethylheptylamino)-1-propenyl)phenyl)methanesulfonamide |
| 3) | (E)-N-(4-(3-(Hexamethyleneimino)-1-propenyl)phenyl)methanesulfonamide |
| 4) | (E)-N-(4-(3-(Dibutylamino)-1-propenyl)phenyl)methanesulfonamide |
| 5) | (E)-N-(4-(4-(Heptamethyleneimino)-1-butenyl)phenyl)methanesulfonamide |
| 6) | (E)-N-(3-(4-(Ethylheptylamino)-1-butenyl)phenyl)methanesulfonamide |
| 7) | (E)-N-(4-(4-(Decylethylamino)-1-butenyl)phenyl)methanesulfonamide |
| 8) | (E)-N-(4-(3-(4-(4-pyridinyl)-1-piperazinyl)-1-propenyl)phenyl)methanesulfonamide |
| 9) | (E)-N-(4-(4-(Hexamethyleneimino)-1-pentenyl)phenylmethanesulfonamide |
| 10) | (E)-N-(4-(4-(dibutylamino)-1-pentenyl)phenyl)methanesulfonamide. |

### Example 5 N-(4-(4-(Ethyl(6-hydroxyheptyl)amino)-1-hydroxybutyl)phenyl)methanesulfonamide

Step I. A solution of 2-methylcyclohexanone (11.1 g, 0.099 mol) in chloroform (15 ml) was added during 20 minutes, under nitrogen, to a stirred suspension of m-chloroperbenzoic acid (24.6 g, 0.143 mol) in chloroform (250 ml). After 3 hours, 40 minutes, the mixture was poured into aqueous sodium bicarbonate and extracted with methylene chloride. The extract was washed with brine, dried (MgSO₄) and concentrated. The residue was distilled from a small amount of K₂CO₃ to give 9.58 g, bp 78-79°C (2.5-3 mm Hg) of 6-hydroxyheptanoic acid, ε-lactone.
Step II. A stirred suspension of dry ethylamine hydrochloride (3.26 g, 0.04 mol) in toluene, under nitrogen, was cooled in an ice bath and treated during 40 minutes with 20 ml of a 2.0 M solution of trimethylaluminum in hexane. The mixture was kept at 0° for 10 minutes and at ambient temperature for 2.5 hours. A portion of the resulting solution (46.8 ml) was added, under nitrogen, during 15 minutes to a solution of the product from Step I (2.0 g, 0.016 mol) in toluene (100 ml). This mixture was warmed at 80°C for 3 hours, cooled and added continuously to dilute HCl. The product was extracted with ethyl acetate. The extracts were dried (MgSO₄) and concentrated to give 2.27 g of N-ethyl-6-hydroxyheptanamide.
Step III. A stirred suspension of LiAlH₄ (2.65 g, 0.0697 mol) in THF (60 ml), under nitrogen, was cooled in an ice bath and treated during 20 minutes with a solution of the product from Step II(4.6 g, 0.0266 mol) in THF (60 ml). The mixture was warmed to ambient temperature during 30 minutes and then refluxed gently for 3.5 hours. It was again cooled in an ice bath and treated cautiously first with H₂O (6 ml) and then with 2.5 N NaOH (5.1 ml). This mixture was stirred at ambient temperature for 1 hour and filtered. The filtrate was concentrated and crystallized from hexane to give 2.63 g of ethyl(6-hydroxyheptyl)amine, mp 37-38°C. The analytical sample had mp 39-41°C. Anal. calcd for C₉H₂₁NO: C, 67.87; H, 13.29; N, 8.80. Found: C, 67.69; H, 13.45; N, 8.81.
Step IV. A stirred solution of 4-((methanesulfonyl)amino)-γ-oxobenzenebutanoic acid (as described in EP 164 865) (0.49 g, 1.8 mmol) in THF (15 ml), under nitrogen, was treated with triethylamine (0.28 ml), cooled to -8°C and treated during 5 minutes with isobutyl chloroformate (0.26 ml, 2.04 mmol). This mixture was kept at -5 to -8°C for 90 minutes and then treated during 30 minutes with a solution of the product from Step III (0.31 g, 1.95 mmol) and triethyl amine (0.28 ml) in THF (10 ml). The mixture was kept at -8°C for 2 hours and then poured into 1 N HCl (19.2 ml). The product was extracted with EtOAc; the extract was washed successively with water, aqueous NaHCO₃, water and brine; dried (MgSO₄) and concentrated. The residue was crystallized from EtOAc-hexane to give 0.36 g (48.6%) of N-ethyl-N-(6-hydroxyheptyl)-γ-oxo-4-((methanesulfonyl)amino)benzenebutanamide, mp 78-80°C.
Step V. A solution of the product from Step IV (1.34 g, 3.28 mmol) in THF (25 ml) was added during 45 minutes under nitrogen, to an ice cold, stirred suspension of LiAlH₄ (0.31 g, 8.2 mmol) in THF (10 ml). The mixture was kept in the ice bath for 90 minutes and then treated cautiously with a solution of saturated aqueous sodium potassium tartrate (6.5 ml) and water (6.5 ml). The mixture was stirred for 90 minutes in the ice bath and then extracted with EtOAc. The extract was washed with brine, dried (MgSO₄) and concentrated; the residue was chromatographed over silica gel with 1% NH₄OH-10% MeOH-CHCl₃ to give 0.72 g of the titled product which is a compound of Formula I'. The high resolution FAB mass spectrum had (M + H)⁺ at m/z 401. Theory for C₂₀H₃₇N₂O₄S: 401.24; measured: 401.2480.

### Example 6 (E)-N-(4-(4-(Ethyl(6-hydroxyheptyl)amino)-1-butenyl)phenyl)methanesulfonamide

In the process as described in Example 2 the product of Example 5 is treated with trifluoroacetic acid to give the titled compound which is a compound of Formula I.

### Example 7 N-(4-(4-(Ethyl(2-cyclohexylethyl)amino)-1-hydroxybutyl)phenyl)methanesulfonamide

Step I. A stirred suspension of 4-((methanesulfonyl)amino)-γ-oxobenzenebutanoic acid (described in EP 164 865) (20 g, 0.0737 mol) in THF (600 ml) was treated with 13.7 ml (0.098 mol) of triethylamine and cooled to -12 °C in an ice-methanol bath. This mixture was treated dropwise with isobutyl chloroformate (12.7 ml, 0.098 mol) and kept at -12°C for 1.5 hours. A solution of ethylamine (4 g, 0.089 mol) and triethylamine (13.7 ml, 0.098 mol) in THF (173 ml) was then added dropwise. The mixture was kept at -12°C for 3 hours and poured into 780 ml of ice-cold 1 N HCl. Nitrogen was bubbled through this mixture to remove the THF. The solid was collected by filtration washed with aqueous NaHCO₃ and water and dried in vacuo to give 14.27 g of crude product. Additional product (4 g) was obtained by extracting the acid filtrate with EtOAc. The combined product was washed with MeOH and dried to give 13.75 g of N-ethyl-γ-oxo-4-((methanesulfonyl)amino)benzenebutanamide. The analytical sample was recrystallized from acetonitrile and had mp 210-213°C. Anal. calcd for C₁₃H₁₈N₂O₄S: C, 52.34; H, 6.08; N, 9.39; S, 10.75. Found: C, 52.02; H, 6.26; N, 9.28; S, 10.63.
Step II. The product from Step I (3.0 g, 0.010 mol) was added in small portions, under nitrogen to a stirred, ice-old mixture of LiAlH₄ (1.15 g, 0.030 mol) in THF (75 ml). This mixture was kept in the ice bath for 1 hour and at ambient temperature for 2 hours. It was then mixed with an additional 100 ml of THF, refluxed for a few minutes and kept at ambient temperature for 18 hours. The mixture was treated carefully with 69 ml of a saturated sodium potassium tartrate solution and stirred for 1 hour. It was extracted with EtOAc. The extract was washed with a dilute sodium chloride solution. The aqueous layer contained the product; it was concentrated and finally freeze-dried. The resulting solid was extracted with MeOH. The methanol solution was concentrated and the residue extracted with CH₂Cl₂. The CH₂Cl₂ solution was concentrated to give 1.6 g of crude product that was purified by chromatography on silica gel with 1% NH₄OH-10 to 20% MeOH-CHCl₃. The product gas crystallized from MeOH-EtOAc to give 540 mg of N-(4-(4-(ethylamino)-1-hydroxybutyl)phenyl)methanesulfonamide,mp 174-176°C. The analytical sample was crystallized from MeOH and had mp 178.5-180.5°C. Anal. calcd for C₁₃H₂₂N₂O₃S: C, 54.52; H, 7.74; N, 9.78; S, 11.20. Found: C, 54.40; H, 7.84; N, 10.00; S, 11.02.
Step III. A stirred solution of cyclohexylacetic acid (2.26 g, 0.0159 mol) and triethylamine (2.28 ml, 0.0163 mol) in THF (120 ml) was cooled to -8°C and treated, dropwise, with isobutyl chloroformate (2.12 ml, 0.0163 mol). The mixture was kept at -8°C for 1.5 hours and then treated with a mixture of the product from Step II (4.0 g, 0.014 mol) and triethylamine (2.28 ml, 0.0163 mol) in THF (160 ml). It was stirred at -8°C for 2 hours, mixed with 1 N HCl (158 ml) and extracted with EtOAc. The extract was washed with water and brine, dried (MgSO₄) and concentrated. The residue was chromatographed on silica gel with 2 to 6% MeOH-CHCl₃ to give 0.654 g of N-(4-(4-(ethylcyclohexylacetyl)amino)-1-hydroxybutyl)phenyl)methanesulfonamide. The mass spectrum had m/z 410 (M⁺).
Step IV. A solution of 1 M LiAlH₄ in THF (3.33 ml) was added, under nitrogen, to 3.33 ml of THF and the stirred solution was cooled in an ice bath and treated during 45 minutes with a solution of the product from Step III (654 mg, 0.00159 mol) in THF (6.42 ml). The mixture was kept in the ice bath for 1 hour 15 minutes and treated cautiously with a saturated aqueous solution of potassium sodium tartrate (3.37 ml). This mixture was extracted with EtOAc; the extract was washed with water and brine, dried (MgSO₄) and concentrated. The residue was chromatographed on silica gel with 3-10% MeOH-CHCl₃. A solution of the product in Et₂O was washed with NaHCO₃, dried (MgSO₄) and concentrated to give 185 mg of the titled compound, a compound of Formula I'. The mass spectrum of this compound had m/z 396 (M⁺).

### Example 8 (E)-N-(4-(4-(Ethyl(2-cyclohexylethyl)amino)-1-butenyl)phenyl)methanesulfonamide

In the process as described in Example 2 the product of Example 7 is treated with trifluoroacetic acid to give the titled compound which is a compound of Formula I.

### Example 9 N-(4-(4-(Ethyl(2-cyclopentylethyl)amino)-1-hydroxybutyl)phenyl)methanesulfonamide

Step I. A stirred solution of cyclopentylacetic acid (2 ml, 0.0159 mol) and triethylamine (2.28 ml, 0.0163 mol) in THF (120 ml) was cooled to -8°C and treated, dropwise with isobutyl chloroformate (2.12 ml, 0.0163 mol). The mixture was kept at -8°C for 1.5 hour and then treated with a mixture of the product from Example 7, Step II, (4.0 g, 0.014 mol) and triethylamine (2.28 ml, 0.0163 mol) in THF (160 ml). It was kept at - 8°C for 1.5 hour and then treated slowly with 1 N HCl (158 ml). The product was extracted with EtOAc. The extract was washed successively with water, saturated aqueous NaHCO₃ and brine, dried (MgSO₄) and concentrated. The residue was chromatographed on silica gel with 5% MeOH-CH₂Cl₂ to give 3.0 g of N-(4-(4-(ethyl(cyclopentylacetyl)amino)-1-hydroxybutyl)phenyl)methanesulfonamide. The mass spectrum had m/z 396 M⁺).
Step II. A 1 M solution of LiAlH₄ in THF (15.9 ml) was mixed with THF (15.9 ml) and cooled, under nitrogen in an ice bath. To this solution was added, dropwise, with stirring, a solution of the product from Step I (3.0 g, 0.0076 mol) in THF (30.7 ml). The mixture was kept in the ice bath for 1 hour and then treated cautiously with a saturated potassium sodium tartrate solution (16.1 ml). This mixture was stirred at ambient temperature for 45 minutes and extracted with EtOAc. The extract was washed with water, dried (MgSO₄) and concentrated. The residue was chromatographed on silica gel with 10-20% MeOH-CHCl₃. A solution of the product in Et₂O was washed with NaHCO₃ and concentrated to give 1.86 g of the titled compound which is a compound of Formula I'. The mass spectrum had m/z 382 (M⁺). Theory for C₂₀H₃₄N₂O₃S: 382.2290; measured: 382.2291.

### Example 10 (E)-N-(4-(4-(Ethyl(2-cyclopentylethyl)amino)-1-butenyl)phenyl)methanesulfonamide

In the process as described in Example 2 the product of Example 9 is treated with trifluoroacetic acid to give the titled compound which is a compound of Formula I.

### Example 11 N-(4-(4-(Ethyl(4,4-dimethylpentyl)amino)-1-hydroxybutyl)phenyl)methanesulfonamide (Intermediate for Example 12)

Step I. A stirred solution of 4,4-dimethylpentanoic acid (2.07 g, 0.0159 mol) and triethylamine (2.28 ml, 0.0163 mol) in THF (120 ml) was cooled to -8°C and treated dropwise with isobutyl chloroformate (2.12 ml, 0.0163 mol). The mixture was kept at - 8°C for 1.5 hour and treated with a mixture of the product from Example 7 Step II (4.0 g, 0.014 mol), triethylamine (2.28 ml, 0.0163 mol) and THF (160 ml). This mixture was kept at -8°C for 1.5 hour and then treated slowly with 1 N HCl (158 ml) and extracted with EtOAc. The extract was washed with water, dilute NaHCO₃ and water, dried (MgSO₄) and concentrated to give 4.4 g of N-(4-(4-(ethyl(4,4-dimethylpentanoyl)amino)-1-hydroxybutyl)phenyl)methanesulfonamide. The mass spectrum had m/z 398 (M⁺).
Step II. A solution of the product from Step I (4.4 g, 0.0115 mol) in THF (46.4 ml) was added dropwise during 45 minutes, under nitrogen to a stirred, ice cold mixture of a 1 M solution of LiAlH₄ in THF (24.0 ml) and THF (24 ml). The mixture was kept in the ice bath for 45 minutes and then treated slowly with a saturated aqueous solution of potassium sodium tartrate (24.4 ml). This mixture was stirred for 30 minutes and extracted with EtOAc. The extracts were washed with water and brine, dried (MgSO₄) and concentrated. The residue was chromatographed on silica gel with 5-10% MeOH-CHCl₃. The product was dissolved in Et₂O, washed with NaHCO₃, dried (MgSO₄) and concentrated to give 2.96 g of the titled compound. The FAB mass spectrum had m/z 385 (M + H)⁺. Theory for C₂₀H₃₇N₂O₃S: 385.2525; measured: 385.2546.

### Example 12 (E)-N-(4-(4-(Ethyl(4,4-dimethylpentyl)amino)-1-butenyl)phenyl)methanesulfonamide

In the process as described in Example 2 the product of Example 11 is treated with trifluoroacetic acid to give the titled compound which is a compound of Formula I.

### Example 13 N-(4-(4-(Ethyl(6-acetoxy-6-methylheptyl)amino)-1-hydroxybutyl)phenyl)methanesulfonamide

Step I. A stirred solution of pentamethylene chlorohydrin (10.0 g, 0.0816 mol) in Et₂O (165 ml) under nitrogen was treated with 3,4-dihydro-2H-pyran (10.3 g, 0.122 mol) and p-toluenesulfonic acid hydrate (0.5 g) and kept at ambient temperature for 4.5 hours. The mixture was washed with aqueous NaHCO₃ and brine, dried (MgSO₄) and concentrated. The residue was distilled to give 4.06 g, bp 79-82°C (0.1-0.07 mm Hg) and 10.54 g, bp 82-84°C (0.1-0.07 mm Hg) of 5-chloropentyl 2-tetrahydropyranyl ether.
Step II. A small portion of a solution of the product from Step I (21.1 g, 0.102 mol) in THF (105 ml) was added, under nitrogen, to magnesium turnings (5.0 g, 0.204 g-atom). The mixture was warmed in an oil bath at 75-80° and the reaction was started by the addition of 1.5 ml of a 1 M solution of 1,2-dibromoethane in THF. The remaining chloroakane solution was then added during 20 minutes. The resulting mixture was refluxed for 45 minutes, cooled in an ice bath and treated during 15 minutes with a solution of acetone (9.0 ml, 0.123 mol) in THF (95 ml). It was kept at ambient temperature for 16 hours, cooled in an ice bath and treated during 15 minutes with saturated aqueous NH₄Cl (115 ml). The resulting mixture was extracted with Et₂O. The extract was washed with water and brine, dried (MgSO₄) and concentrated to give 30.5 g of crude product. Distillation gave 16.77g of 6-hydroxy-6-methylheptyl 2-tetrahydropyranyl ether, bp 107-115° (0.07-0.1 mm Hg). The CI mass spectrum had m/z 231 (M+H)⁺.
Step III. A solution of the product from Step II (5.0 g, 0.0217 mol) in triethylamine (6.1 ml, 0.0434 mol), under nitrogen, was treated with acetic anhydride (4.1 ml, 0.434 mol) and 4-dimethylaminopyridine (0.27 g, 0.00217 mol). It was kept at ambient temperature for 20.5 hours, diluted with hexane (100 ml), washed successively with 75 ml of cold 5% HCl, aqueous NaHCO₃ and brine, dried (MgSO₄) and concentrated. The residue was chromatographed on silica gel with 0.05% Et₃N-5% EtOAc-hexane to give 4.92 g of 6-acetoxy-6-methylheptyl 2-tetrahydropyranyl ether. The CI mass spectrum had m/z 273 (M+H)⁺.
Step IV. A stirred solution of the product from Step III (4.83 g, 0.0177 mol) in absolute EtOH (150 ml) was treated with pyridinium p-toluenesulfonate (0.58 g, 0.0023 mol), kept at ambient temperature for 46 hours and concentrated. The residue was dissolved in Et₂O, washed with aqueous NaHCO₃ and brine, dried (MgSO₄) and concentrated. The resulting oil was chromatographed on silica gel with 0.05% Et₃N-5 to 20% EtOAc-hexane to give 2.88 g of 6-acetoxy-6-methyl-1-heptanol.
Step V. A stirred solution of the product from Step IV (2.79 g, 0.0148 mol) in benzene (27 ml), under nitrogen, was treated with triphenylphosphine (4.27 g, 0.0163 mol), cooled in an ice bath and treated, portion wise during 26 minutes, with N-bromosuccinimide (2.90 g, 0.0163 mol). The mixture was kept in the ice bath for 30 minutes and at ambient temperature for 4.5 hours. It was then mixed with pentane (100 ml). The solid was collected by filtration and washed with pentane. The filtrate was concentrated; the residue was again mixed with pentane and filtered. This filtrate was mixed with a little Et₂O, washed successively with cold 5% aqueous sodium thiosulfate, 0.5 N NaOH and brine, dried (MgSO₄) and concentrated. The residue was chromatographed on silica gel with 1.5-2% EtOAc-hexane to give 3.25 g of 6-acetoxy-1-bromo-6-methylheptane.
Step VI. A stirred mixture of the product from Example 7, Step II (1.5 g, 0.00524 mol), the product from Step V above 1.45 g, 0.00576 mol), sodium bicarbonate (0.88 g, 0.0105 mol) and acetonitrile (45 ml) was warmed at 90-95°, under nitrogen for 6 hours and kept at ambient temperature for 11 hours. It was then filtered. The filtrate was concentrated and the residue chromatographed on silica gel with 0.5% NH₄OH-6% MeOH-CH₂Cl₂ to give 1.63 g of the titled compound which is a compound of Formula I'. The mass spectrum had m/z 456 (M⁺).

### Example 14 (E)-N-(4-(4-(Ethyl(6-acetoxy-6-methylheptyl)amino)-1-butenyl)phenyl)methanesulfonamide

In the process as described in Example 2 the product from Example 13 is treated with trifluoroacetic acid to give the titled compound which is a compound of Formula I.

### Example 15 N-(4-(4-(Ethyl(6-hydroxy-6-methylheptyl)amino)-1-hydroxybutyl)phenyl)methanesulfonamide

A stirred solution of the product from Example 13 (1.35 g, 0.00296 mol) in methanol (80 ml) was treated with a solution of K₂CO₃ (2.04 g, 0.0148 mol) in water (5.9 ml) and the mixture was refluxed for 21.5 hours. It was kept at ambient temperature for 42.5 hours and concentrated to an aqueous residue which was mixed with water (10 ml) and CH₂Cl₂, acidified to pH 8.5-9 with 6 NHCl, saturated with NaCl and extracted with CH₂Cl₂. The extract was dried (MgSO₄), concentrated and the residue chromatographed on silica gel with 0.5% NH₄OH-9 % MeOH-CH₂Cl₂ to give 1.1 g of the titled compound which is a compound of Formula I'.

### Example 16 (E)-N-(4-(4-(Ethyl(6-hydroxy-6-methylheptyl)amino)-1-butenyl)phenyl)methanesulfonamide

In the process as described in Example 2 the product from Example 15 is treated with trifluoroacetic acid to give the titled compound which is a compound of Formula I.

### Example 17 N-(4-(4-(Ethyl(6-fluoro-6-methylheptyl)amino)-1-hydoxybutyl)phenyl)methanesulfonamide

Step I. A solution of the product from Example 13, Step II, (3.97 g, 0.0173 mol) in CH₂Cl₂ (12 ml) was added under nitrogen, during 4.5 minutes to a stirred solution of diethylaminosulfur trifluoride (4.6 ml, 0.0345 mol) in CH₂Cl₂ (12 ml) that had been cooled in a dry ice acetone bath (-78°C). The mixture was kept in the bath for 15 minutes, warmed to 0° during 10 minutes and mixed with 10% aqueous Na₂CO₃ (60 ml). This mixture was extracted with CH₂Cl₂. The extracts were washed with water, dried (MgSO₄) and concentrated. The residue was chromatographed on silica gel with 0.05% Et₃N-2.5% EtOAc-hexane to give 3.36 g of 6-fluoro-6-methylheptyl 2-tetrahydropyranyl ether.
Step II. A stirred solution of the product from Step I (3.34 g, 0.0144 mol) in absolute EtOH was treated with pyridinium p-toluenesulfonate (0.47 g, 0.00187 mol) and kept under nitrogen at ambient temperature for 41 hours. The mixture was concentrated and the residue, dissolved in EtOAc, was washed with aqueous NaHCO₃ and brine, dried (MgSO₄) and concentrated. The residue was chromatographed on silica gel with 5 to 20% EtOAc-hexane to give 1.86 g of 6-fluoro-6-methyl-1-heptanol.
Step III. A stirred solution of the product from Step II (0.427 g, 0.00288 mol) in benzene (5.2 ml) was mixed with triphenylphosphine (0.83 g, 0.00317 mol) cooled in an ice bath and treated, portion-wise, during 26 minutes with N-bromosuccinimide (0.56 g, 0.00317 mol). The mixture was kept in the ice bath for 30 minutes and at ambient temperature for 3.5 hours; it was then diluted with pentane (20 ml), cooled in an ice bath for a few minutes and filtered. The solid was washed with pentane and the filtrate was concentrated. A mixture of the residue and pentane was cooled in an ice bath for a few minutes and again filtered. The filtrate was mixed with Et₂O and washed successively with cold 5% aqueous sodium thiosulfate, 0.5 N NaOH and brine, dried (MgSO₄) and concentrated. The residue was chromatographed on silica gel with 1-3% EtOAc-hexane to give 0.440 g of 1-bromo-6-fluoro-6-methylheptane.
Step IV. A stirred mixture of the product from Example 7, Step II, (0.37 g, 0.00128 mol), the product from Step III above (0.298 g, 0.0141 mol), sodium bicarbonate (0.21 g, 0.0026 mol) and acetonitrile (11 ml) was refluxed for 5 hours and kept at ambient temperature for 18 hours. It was filtered and the solid was washed with acetonitrile. The filtrate was concentrated and the residue was chromatographed on silica gel with 10% MeOH-CH₂Cl₂ to give 0.332 g of the titled product, a compound of Formula I'. The mass spectrum had m/z 416 (M⁺).

### Example 18 (E)-N-(4-(4-(Ethyl(6-fluoro-6-methylheptyl)amino)-1-butenyl)phenyl)methanesulfonamide

In the process as described in Example 2 the product from Example 17 is treated with trifluoroacetic acid to give the titled compound which is a compound of Formula I..

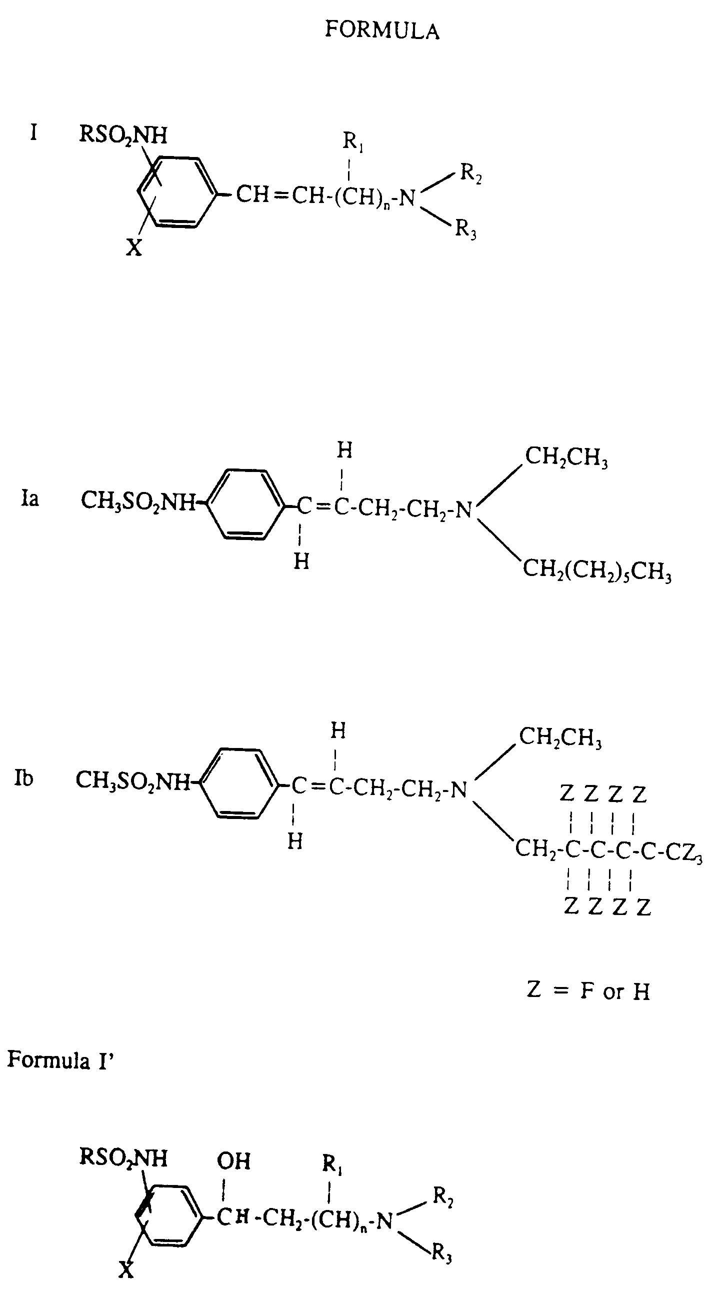

## Claims (Claims for the following Contracting State(s): AT, BE, CH, DE, DK, FR, GB, IT, LI, LU, NL, SE)

1. A compound of formula I 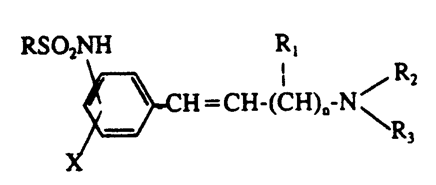 or a pharmacologically-acceptable salt thereof, wherein:
n is 1 to 3;
X is hydrogen, hydroxy, C₁₋₄ alkoxy, C₁₋₄ alkyl, CF₃ or halogen;
R is C₁₋₄ alkyl;
R₁ is hydrogen or C₁₋₄ alkyl; and
either R₂ is C₁₋₁₀ alkyl and R₃ is
a) C₁₋₁₀ alkyl,
b) C₁₋₇ alkyl substituted by aryl, heteroaryl or C₃₋₇ cycloalkyl,
c) C₁₋₁₀ alkyl substituted by 1 to 8 F atoms,
d) C₃₋₁₀ cycloalkyl,
e) C₃₋₁₀ alkenyl, or
f) C₁₋₁₀ alkyl substituted with one to three hydroxy, C₁₋₄ acyloxy or C₁₋₅ alkoxy substituents,
provided that the sum of carbon atoms in R₂ and R₃ is greater than five; or
NR₂R₃ is a saturated heterocyclic group having one nitrogen atom and 4-8 carbon atoms or is 4-substituted piperazine in which the 4-substituent is C₁₋₁₀ alkyl, aryl, benzyl or heteroaryl.

2. A compound of claim 1, wherein X is hydrogen.

3. A compound of claim 1 or claim 2, wherein R₁ is hydrogen.

4. A compound of any preceding claim, wherein only one occurrence of R₁ is alkyl.

5. A compound of any preceding claim, wherein R₃ is C₁₋₁₀ alkyl substituted by 1 to 8 F atoms.

6. A compound of claim 1, which is:
(E)-N-(4-(4-(ethylheptylamino)-1-butenyl)phenyl)methanesulfonamide;
(E)-N-(4-(4-(hexahydro-1H-azepin-1-yl)-1-butenyl)phenyl) methanesulfonamide;
(E)-N-(4-(4-(dibutylamino)-1-butenyl)phenyl)methanesulfonamide;
(E)-N-(4-(4-(dipropylamino)-1-butenyl)phenyl)isopropanesulfonamide;
(E)-N-(4-(4-(heptamethyleneimino)-1-butenyl)phenyl)methanesulfonamide;
(E)-N-(3-(4-(ethylheptylamino)-1-butenyl)phenyl)methanesulfonamide;
(E)-N-(4-(4-(decylethylamino)-1-butenyl)phenyl)methanesulfonamide;
(E)-N-(4-(4-(ethyl(6-hydroxyheptyl)amino)-1-butenyl)phenyl)methanesulfonamide;
(E)-N-(4-(4-(ethyl(2-cyclohexylethyl)amino)-1-butenyl)phenyl)methanesulfonamide;
(E)-N-(4-(4-(ethyl(2-cyclopentylethyl)amino)-1-butenyl)phenyl)methanesulfonamide;
(E)-N-(4-(4-ethyl(4,4-dimethylpentyl)amino)-1-butenyl)phenyl)methanesulfonamide;
(E)-N-(4-(4-ethyl(6-acetoxy-6-methylheptyl)amino)-1-butenyl)phenyl)methanesulfonamide; or
(E)-N-(4-(4-ethyl(6-fluoro-6-methylheptyl)amino)-1-butenyl)phenyl)methanesulfonamide.

7. A compound of claim 1, which is:
(E)-N-(4-(3-(ethylheptylamino)-1-propenyl)phenyl)methanesulfonamide;
(E)-N-(4-(3-(hexamethyleneimino)-1-propenyl)phenyl)methanesulfonamide;
(E)-N-(4-(3-(dibutylamino)-1-propenyl)phenyl)methanesulfonamide; or
(E)-N-(4-(3-(4-(4-pyridinyl)-1-piperazinyl)-1-propenyl)phenyl)methanesulfonamide

8. A compound of claim 1, which is:
(E)-N-(4-(hexamethyleneimino)-1-pentenyl)phenyl)methanesulfonamide; or
(E)-N-(4-(dibutylamino)-1-pentenyl)phenyl)methanesulfonamide.

9. Use of a compound of any preceding claim, for the preparation of a medicament for treating cardiac arrhythmia in mammals.

10. Use according to claim 9, wherein the medicament is adapted for oral, sublingual, transdermal or parenteral administration.

11. A compound of formula I' 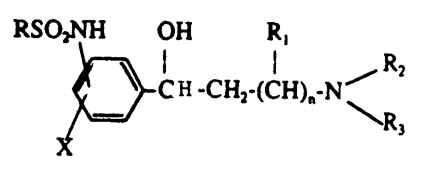 or a pharmacologically-acceptable salt thereof, wherein:
n, X, R and R₁ are as defined in claim 1;
R₂ is C₁₋₁₀ alkyl; and
R₃ is C₁₋₇ alkyl substituted by aryl, heteroaryl or C₃₋₇ cycloalkyl, or C₁₋₁₀ alkyl substituted by 1 to 8 F atoms or one to three hydroxy, C₁₋₅ acyloxy or C₁₋₄ alkoxy substituents, provided that the sum of carbon atoms in R₂ and R₃ is greater than five.

12. A compound of claim 11, wherein R₃ is C₁₋₁₀ alkyl substituted by 1 to 8 F atoms.

13. A compound of claim 11, which is:
N-(4-(4-(ethyl(6-hydroxyheptyl)amino)-1-hydroxybutyl)phenyl)methanesulfonamide;
N-(4-(4-(ethyl(2-cyclohexylethyl)amino)-1-hydroxybutyl)phenyl)methanesulfonamide;
N-(4-(4-(ethyl(6-acetoxy-6-methylheptyl)amino)-1-hydroxybutyl)phenyl)methanesulfonamide;
N-(4-(4-(ethyl(6-hydroxy-6-methylheptyl)amino)-1-hydroxybutyl)phenyl)methanesulfonamide; or
N-(4-(4-(ethyl(6-fluoro-6-methylheptyl)amino)-1-hydroxybutyl)phenyl)methanesulfonamide.

## Claims (Claims for the following Contracting State(s): ES)

1. A process for preparing a compound of formula I 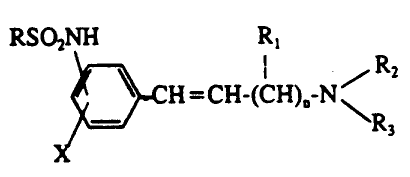 or a pharmacologically-acceptable salt thereof, wherein:
n is 1 to 3;
X is hydrogen, hydroxy, C₁₋₄ alkoxy, C₁₋₄ alkyl, CF₃ or halogen;
R is C₁₋₄ alkyl;
R₁ is hydrogen or C₁₋₄ alkyl; and
either R₂ is C₁₋₁₀ alkyl and R₃ is
a) C₁₋₁₀ alkyl,
b) C₁₋₇ alkyl substituted aryl, heteroaryl or C₃₋₇ cycloalkyl,
c) C₁₋₁₀ alkyl substituted by 1 to 8 F atoms,
d) C₃₋₁₀ cycloalkyl,
e) C₃₋₁₀ alkenyl,
f) C₁₋₁₀ alkyl substituted with one to three hydroxy, C₁₋₄ acyloxy or C₁₋₅ alkoxy substituents,
provided that the sum of carbon atoms in R₂ and R₃ is greater than five; or
NR₂R₃ is a saturated heterocyclic group having one nitrogen atom and 4-8 carbon atoms or is 4-substituted piperazine in which the 4-substituent is C₁₋₁₀ alkyl, aryl, bensyl or heteroaryl,
which comprises dehydrating a corresponding benzylic alcohol.

2. A process of claim 1, wherein X is hydrogen.

3. A process of claim 1 or claim 2, wherein R₁ is hydrogen.

4. A process of any preceding claim, wherein only one occurrence of R₁ is alkyl.

5. A process of any preceding claim, wherein R₃ is C₁₋₁₀ alkyl substituted by 1 to 8 F atoms.

6. A process of claim 1, wherein the compound of formula I is:
(E)-N-(4-(4-(ethylheptylamino)-1-butenyl)phenyl)methanesulfonamide;
(E)-N-(4-(4-(hexahydro-1H-azepin-1-yl)-1-butenyl)phenyl)methanesulfonamide;
(E)-N-(4-(4-(dibutylamino)-1-butenyl)phenyl)methanesulfonamide;
(E)-N-(4(4-(dipropylamino)-1-butenyl)phenyl)isopropanesulfonamide;
(E)-N-(4-(4-(heptamethyleneimino)-1-butenyl)phenyl)methanesulfonamide;
(E)-N-(3-(4-(ethylheptylamino)-1-butenyl)phenyl)methanesulfonamide;
(E)-N-(4-(4-(decylethylamino)-1-butenyl)phenyl)methanesulfonamide;
(E)-N-(4-(4-(ethyl(6-hydroxyheptyl)amino)-1-butenyl)phenyl)methanesulfonamide;
(E)-N-(4-(4-(ethyl(2-cyclohexylethyl)amino)-1-butenyl)phenyl)methanesulfonamide;
(E)-N-(4-(4-(ethyl(2-cyclopentylethyl)amino)-1-butenyl)phenyl)methanesulfonamide;
(E)-N-(4-(4-ethyl(4,4-dimethylpentyl)amino)-1-butenyl)phenyl)methanesulfonamide;
(E)-N-(4-(4-ethyl(6-acetoxy-6-methylheptyl)amino)-1-butenyl)phenyl)methanesulfonamide; or
(E)-N-(4-(4-ethyl(6-fluoro-6-methylheptyl)amino)-1-butenyl)phenyl)methanesulfonamide.

7. A process of claim 1, wherein the compound of formula I is:
(E)-N-(4-(3-(ethylheptylamino)-1-propenyl)phenyl)methanesulfonamide;
(E)-N-(4-(3-(hexamethyleneimino)-1-propenyl)phenyl)methanesulfonamide;
(E)-N-(4-(3-(dibutylamino)-1-propenyl)phenyl)methanesulfonamide; or
(E)-N-(4-(3-(4-(4-pyridinyl)-1-piperazinyl)-1-propenyl)phenyl)methanesulfonamide.

8. A process of claim 1, wherein the compound of formula I is:
(E)-N-(4-(hexamethyleneimino)-1-pentenyl)phenyl)methanesulfonamide; or
(E)-N-(4-(dibutylamino)-1-pentenyl)phenyl)methanesulfonamide.

9. A process according to any preceding claim, wherein the dehydration is conducted by reaction with trifluoroacetic acid in a solvent at 0-40°C.

10. Use of a compound of formula I as defined in any of claims 1 to 8, for the manufacture of a medicament for the treatment of arrhythmia.

## Patentansprüche (Patentansprüche für folgende(n) Vertragsstaat(en): AT, BE, CH, DE, DK, FR, GB, IT, LI, LU, NL, SE)

1. Verbindung der Formel I 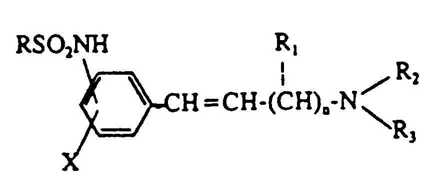 oder ein pharmakologisch akzeptables Salz derselben, wobei bedeuten:
n = 1 bis 3;
X Wasserstoff, Hydroxy, C₁₋₄ Alkoxy, C₁₋₄ Alkyl, CF₃ oder Halogen;
R C₁₋₄ Alkyl;
R₁ Wasserstoff oder C₁₋₄ Alkyl und entweder
R₂ C₁₋₁₀ Alkyl und
R₃
a) C₁₋₁₀ Alkyl,
b) C₁₋₇ Alkyl, substituiert durch Aryl, Heteroaryl oder C₃₋₇ Cycloalkyl,
c) C₁₋₁₀ Alkyl, substituiert durch 1 bis 8 F Atom(e),
d) C₃₋₁₀ Cycloalkyl,
e) C₃₋₁₀ Alkenyl oder
f) C₁₋₁₀ Alkyl, ein- bis dreifach substituiert durch Hydroxy-, C₁₋₄ Acyloxy- oder C₁₋₅ Alkoxysubstituenten,
wobei die Summe der Kohlenstoffatome in R₂ und R₃ 5 übersteigt, oder
NR₂R₃ eine gesättigte heterocyclische Gruppe mit einem Stickstoffatom und 4 bis 8 Kohlenstoffatomen oder 4-substituiertes Piperazin, bei welchem der 4-Substituent aus C₁₋₁₀ Alkyl, Aryl, Benzyl oder Heteroaryl besteht.

2. Verbindung nach Anspruch 1, wobei X für Wasserstoff steht.

3. Verbindung nach Anspruch 1 oder 2, wobei R₁ für Wasserstoff steht.

4. Verbindung nach einem der vorhergehenden Ansprüche, wobei lediglich ein vorhandener Rest R₁ Alkyl ist.

5. Verbindung nach einem der vorhergehenden Ansprüche, wobei R₃ für C₁₋₁₀ Alkyl, substituiert durch 1 bis 8 F Atom(e) steht.

6. Verbindung nach Anspruch 1, nämlich:
(E)-N-(4-(4-(Ethylheptylamino)-1-butenyl)phenyl)-methansulfonamid;
(E)-N-(4-(4-Hexahydro-1H-azepin-1-yl)-1-butenyl)-phenyl)-methansulfonamid,
(E)-N-(4-(4-(Dibutylamino)-1-butenyl)-phenyl)-methansulfonamid;
(E)-N-(4-(4-(Dipropylamino)-1-butenyl)-phenyl)-isopropansulfonamid;
(E)-N-(4-(4-(Heptamethylenimino)-1-butenyl)-phenyl)-methansulfonamid;
(E)-N-(3-(4-(Ethylheptylamino)-1-butenyl)-phenyl)-methansulfonamid;
(E)-N-(4-(4-(Decylethylamino)-1-butenyl)-phenyl)-methansulfonamid;
(E)-N-(4-(4-(Ethyl-(6-hydroxyheptyl)-amino)-1-butenyl)-phenyl)-methansulfonamid;
(E)-N-(4-(4-(Ethyl-(2-cyclohexylethyl)-amino)-1-butenyl)-phenyl)-methansulfonamid;
(E)-N-(4-(4-(Ethyl-(2-cyclopentylethyl)-amino)-1-butenyl)-phenyl)-methansulfonamid;
(E)-N-(4-(4-Ethyl-(4,4-dimethylpentyl)-amino)-1-butenyl)-phenyl)-methansulfonamid;
(E)-N-(4-(4-Ethyl-(6-acetoxy-6-methylheptyl)-amino)-1-butenyl)-phenyl)-methansulfonamid oder
(E)-N-(4-(4-Ethyl-(6-fluor-6-methylheptyl)-amino)-1-butenyl)-phenyl)-methansulfonamid.

7. Verbindung nach Anspruch 1, nämlich:
(E)-N-(4-(3-(ethylheptylamino)-1-propenyl)phenyl)-methansulfonamid;
(E)-N-(4-(3-(Hexamethylenimino)-1-propenyl)-phenyl)-methansulfonamid;
(E)-N-(4-(3-(Dibutylamino)-1-propenyl)-phenyl)-methansulfonamid oder
(E)-N-(4-(3-(4-(4-Pyridinyl)-1-piperazinyl)-1-propenyl)-phenyl)-methansulfonamid.

8. Verbindung nach Anspruch 1, nämlich:
(E)-N-(4-(Hexamethylenimino)-1-pentenyl)-phenyl)-methansulfonamid oder
(E)-N-(4-(Dibutylamino)-1-pentenyl)-phenyl)-methansulfonamid.

9. Verwendung einer Verbindung nach einem der vorhergehenden Ansprüche zur Herstellung eines Medikaments zur Behandlung von Herzrhythmusstörungen bei Säugetieren.

10. Verwendung nach Anspruch 9, wobei das Medikament für eine orale, sublinguale, transdermale oder parenterale Verabreichung vorgesehen ist.

11. Verbindung der Formel I' 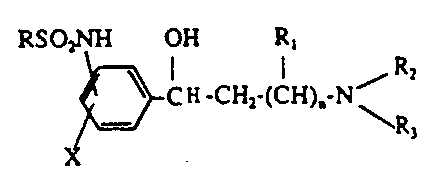 oder ein pharmakologisch akzeptables Salz derselben,
wobei n, X, R und R₁ die in Anspruch 1 angegebene Bedeutung besitzen,
R₂ für C₁₋₁₀ Alkyl steht und
R₃ C₁₋₇ Alkyl, substituiert durch Aryl, Heteroaryl oder C₃₋₇ Cycloalkyl, oder C₁₋₁₀ Alkyl, substituiert durch 1 bis 8 F Atom(e) oder 1 bis 3 Hydroxy-, C₁₋₅ Acyloxy- oder C₁₋₄ Alkoxysubstituenten bedeutet, wobei die Summe der Kohlenstoffatome in R₂ und R₃ 5 übersteigt.

12. Verbindung nach Anspruch 11, wobei R₃ für C₁₋₁₀ Alkyl, substituiert durch 1 bis 8 F Atom(e) steht.

13. Verbindung nach Anspruch 11, nämlich:
N-(4-(4-(Ethyl-(6-hydroxyheptyl)-amino)-1-hydroxybutyl)-phenyl)-methansulfonamid;
N-(4-(4-(Ethyl-(2-cyclohexylethyl)-amino)-1-hydroxybutyl)-phenyl)-methansulfonamid;
N-(4-(4-(Ethyl-(6-acetoxy-6-methylheptyl)-amino)-1-hydroxybutyl)-phenyl)-methansulfonamid;
N-(4-(4-(Ethyl-(6-hydroxy-6-methylheptyl)-amino)-1-hydroxybutyl)-phenyl)-methansulfonamid oder
N-(4-(4-(Ethyl-(6-fluor-6-methylheptyl)amino)-1-hydroxybutyl)-phenyl)-methansulfonamid.

## Patentansprüche (Patentansprüche für folgende(n) Vertragsstaat(en): ES)

1. Verfahren zur Herstellung einer Verbindung der Formel I 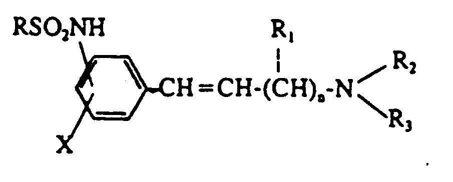 oder eines pharmakologisch akzeptablen Salzes derselben, wobei bedeuten:
n = 1 bis 3;
X Wasserstoff, Hydroxy, C₁₋₄ Alkoxy, C₁₋₄ Alkyl, CF₃ oder Halogen;
R C₁₋₄ Alkyl;
R₁ Wasserstoff oder C₁₋₄ Alkyl und entweder
R₂ C₁₋₁₀ Alkyl und
R₃
a) C₁₋₁₀ Alkyl,
b) C₁₋₇ Alkyl, substituiert durch Aryl, Heteroaryl oder C₃₋₇ Cycloalkyl,
c) C₁₋₁₀ Alkyl, substituiert durch 1 bis 8 F Atom(e),
d) C₃₋₁₀ Cycloalkyl,
e) C₃₋₁₀ Alkenyl oder
f) C₁₋₁₀ Alkyl, ein- bis dreifach substituiert durch Hydroxy-, C₁₋₄ Acyloxy- oder C₁₋₅ Alkoxysubstituenten,
wobei die Summe der Kohlenstoffatome in R₂ und R₃ 5 übersteigt, oder
NR₂R₃ eine gesättigte heterocyclische Gruppe mit einem Stickstoffatom und 4 bis 8 Kohlenstoffatomen oder 4-substituiertes Piperazin, bei welchem der 4-Substituent aus C₁₋₁₀ Alkyl, Aryl, Benzyl oder Heteroaryl besteht, durch Dehydratisieren eines entsprechenden Benzylalkohols.

2. Verfahren nach Anspruch 1, wobei X für Wasserstoff steht.

3. Verfahren nach Anspruch 1 oder 2, wobei R₁ für Wasserstoff steht.

4. Verfahren nach einem der vorhergehenden Ansprüche, wobei lediglich ein vorhandener Rest R₁ Alkyl ist.

5. Verfahren nach einem der vorhergehenden Ansprüche, wobei R₃ für C₁₋₁₀ Alkyl, substituiert durch 1 bis 8 F Atom(e) steht.

6. Verfahren nach Anspruch 1, wobei die Verbindung der Formel I aus:
(E)-N-(4-(4-(Ethylheptylamino)-1-butenyl)-phenyl)-methansulfonamid;
(E)-N-(4-(4-Hexahydro-1H-azepin-1-yl)-1-butenyl)-phenyl)-methansulfonamid;
(E)-N-(4-(4-(Dibutylamino)-1-butenyl)-phenyl)-methansulfonamid;
(E)-N-(4-(4-(Dipropylamino)-1-butenyl)-phenyl)-isopropansulfonamid;
(E)-N-(4-(4-(Heptamethylenimino)-1-butenyl)-phenyl)-methansulfonamid;
(E)-N-(3-(4-(Ethylheptylamino)-1-butenyl)-phenyl)-methansulfonamid;
(E)-N-(4-(4-(Decylethylamino)-1-butenyl)-phenyl)-methansulfonamid;
(E)-N-(4-(4-(Ethyl-(6-hydroxyheptyl)-amino)-1-butenyl)-phenyl)-methansulfonamid;
(E)-N-(4-(4-(Ethyl-(2-cyclohexylethyl)-amino)-1-butenyl)-phenyl)-methansulfonamid;
(E)-N-(4-(4-(Ethyl-(2-cyclopentylethyl)-amino)-1-butenyl)-phenyl)-methansulfonamid;
(E)-N-(4-(4-Ethyl-(4,4-dimethylpentyl)-amino)-1-butenyl)-phenyl)-methansulfonamid;
(E)-N-(4-(4-Ethyl-(6-acetoxy-6-methylheptyl)-amino)-1-butenyl)-phenyl)-methansulfonamid oder
(E)-N-(4-(4-Ethyl-(6-fluor-6-methylheptyl)-amino)-1-butenyl)-phenyl)-methansulfonamid
besteht.

7. Verfahren nach Anspruch 1, wobei die Verbindung der Formel I aus:
(E)-N-(4-(3-(Ethylheptylamino)-1-propenyl)-phenyl)-methansulfonamid;
(E)-N-(4-(3-(Hexamethylenimino)-1-propenyl)-phenyl)-methansulfonamid;
(E)-N-(4-(3-(Dibutylamino)-1-propenyl)-phenyl)-methansulfonamid oder
(E)-N-(4-(3-(4-(4-Pyridinyl)-1-piperazinyl)-1-propenyl)-phenyl)-methansulfonamid
besteht.

8. Verfahren nach Anspruch 1, wobei die Verbindung der Formel I aus:
(E)-N-(4-(Hexamethylenimino)-1-pentenyl)-phenyl)-methansulfonamid oder
(E)-N-(4-(Dibutylamino)-1-pentenyl)-phenyl)-methansulfonamid
besteht.

9. Verfahren nach einem der vorhergehenden Ansprüche, wobei die Dehydratisierung durch Umsetzen mit Trifluoressigsäure in einem Lösungsmittel bei 0 - 40°C durchgeführt wird.

10. Verwendung einer Verbindung der Formel I gemäß der Definition nach einem der Ansprüche 1 bis 8 zur Herstellung eines Medikaments zur Behandlung von Rhythmusstörungen.

## Revendications (Revendications pour l'(les) Etat(s) contractant(s) suivant(s): AT, BE, CH, DE, DK, FR, GB, IT, LI, LU, NL, SE)

1. Composé de formule I 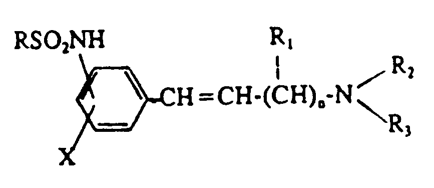 ou un sel pharmacologiquement acceptable de ce composé, formule dans laquelle :
n a une valeur de 1 à 3 ;
X représente l'hydrogène, un groupe hydroxy, alkoxy en C₁ à C₄, alkyle en C₁ à C₄, CF₃ ou un halogène :
R est un groupe alkyle en C₁ à C₄ ;
R₁ est de l'hydrogène ou un groupe alkyle en C₁ à C₄ : et
R₂ est un groupe alkyle en C₁ à C₁₀ et R₃ représente
a) un groupe alkyle en C₁ à C₁₀,
b) un groupe alkyle en C₁ à C₇ substitué par un radical aryle, hétéroaryle ou cycloalkyle en C₃ à C₇,
c) un groupe alkyle en C₁ à C₁₀ substitué par 1 à 8 atomes de fluor,
d) un groupe cycloalkyle en C₃ à C₁₀,
e) un groupe alcényle en C₃ à C₁₀, ou
f) un groupe alkyle en C₁ à C₁₀ substitué par 1 à 3 substituants hydroxy, acyloxy en C₁ à C₄ ou alkoxy en C₁ à C₅,
sous réserve que la somme des atomes de carbone dans R₂ et R₃ soit supérieure à 5 ; ou bien
NR₂R₃ est un groupe hétérocyclique saturé ayant un atome d'azote et 4 à 8 atomes de carbone ou est une piperazine substituée en position 4 dans laquelle le substituant en position 4 est un groupe alkyle en C₁ à C₁₀, aryle, benzyle ou hétéroaryle.

2. Composé suivant la revendication 1, dans lequel X est de l'hydrogène.

3. Composé suivant la revendication 1 ou la revendication 2, dans lequel R₁ est de l'hydrogène.

4. Composé suivant l'une quelconque des revendications précédentes, dans lequel R₁ n'apparaît qu'une seule fois sous forme d'un groupe alkyle.

5. Composé suivant l'une quelconque des revendications précédentes, dans lequel R₃ est un groupe alkyle à C₁ à C₁₀ substitué par 1 à 8 atomes de fluor.

6. Composé suivant la revendication 1, qui est :
le (E)-N-(4-(4-(éthylheptylamino)-1-butényl)phényl)méthanesulfonamide ;
le (E)-N-(4-(4-(hexahydro-1H-azépine-1-yl)-1-butényl)phényl)méthanesulfonamide ;
le (E)-N-(4-(4-(dibutylamino)-1-butényl)phényl)méthanesulfonamide ;
le (E)-N-(4-(4-(dipropylamino)-1-butényl)phényl)isopropanesulfonamide ;
le (E)-N-(4-(4-(heptaméthylène-imino)-1-butényl)phényl)méthanesulfonamide ;
le (E)-N-(3-(4-(éthylheptylamino)-1-butényl)phényl)méthanesulfonamide ;
le (E)-N-(4-(4-(décyléthylamino)-1-butényl)phényl)méthanesulfonamide ;
le (E)-N-(4-(4-(éthyl(6-hydroxyheptyl)amino)-1-butényl)phényl)méthanesulfonamide ;
le (E)-N-(4-(4-(éthyl(2-cyclohexyléthyl)amino)-1-butényl)phényl)méthanesulfonamide ;
le (E)-N-(4-(4-(éthyl(2-cyclopentyléthyl)amino)-1-butényl)phényl)méthanesulfonamide ;
le (E)-N-(4-(4-(éthyl(4,4-diméthylpentyl)amino)-1-butényl)phényl)méthanesulfonamide ;
le (E)-N-(4-(4-éthyl(6-acétoxy-6-méthylheptyl)amino)-1-butényl)phényl)méthanesulfonamide ; ou
le (E)-N-(4-(4-(éthyl(6-fluoro-6-méthylheptyl)amino)-1-butényl)phényl)méthanesulfonamide.

7. Composé suivant la revendication 1, qui est :
le (E)-N-(4-(3-(éthylheptylamino)-1-propényl)phényl)méthanesulfonamide ;
le (E)-N-(4-(3-(hexaméthylène-imino)-1-propényl)phényl)méthanesulfonamide ;
le (E)-N-(4-(3-(dibutylamino)-1-propényl)phényl)méthanesulfonamide ; ou
le (E)-N-(4-(3-(4-(4-pyridinyl)-1-pipérazinyl-1-propényl)phényl)méthanesulfonamide.

8. Composé suivant la revendication 1, qui est :
le (E)-N-(4-hexaméthylène-imino)-1-pentényl)phényl)méthanesulfonamide ; ou
le (E)-N-(4-(dibutylamino)-1-pentényl)phényl)méthanesulfonamide.

9. Utilisation d'un composé suivant l'une quelconque des revendications précédentes pour la préparation d'un médicament destiné au traitement de l'arythmie cardiaque chez des mammifères.

10. Utilisation suivant la revendication 9, dans laquelle le médicament est adapté à l'administration orale, sublinguale, transdermique ou parentérale.

11. Composé de formule I' 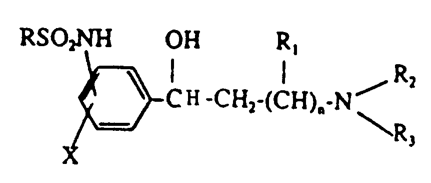 ou d'un sel pharmaceutiquement acceptable de ce composé, formule dans laquelle :
ni X, R et R₁ sont tels que définis dans la revendication 1 ;
R₂ est un groupe alkyle en C₁ à C₁₀ ; et
R₃ est un groupe alkyle en C₁ à C₇ substitué par un radical aryle, hétéroaryle ou cycloalkyle en C₃ à C₇, ou un groupe alkyle en C₁ à C₁₀ substitué par 1 à 8 atomes de fluor ou par 1 à 3 substituants hydroxy, acyloxy en C₁ à C₅ ou alkoxy en C₁ à C₄, sous réserve que la somme des atomes de carbone dans R₂ et R₃ soit supérieure à 5.

12. Composé suivant la revendication 11, dans lequel R₃ est un groupe alkyle en C₁ à C₁₀ substitué par 1 à 8 atomes de fluor.

13. Composé suivant la revendication 11, qui est :
le N(4-(4-(éthyl(6-hydroxyheptyl)amino)-1-hydroxybutyl)phényl)méthanesulfonamide ;
le N-(4-(4-(éthyl(2-cyclohexyléthyl)amino)-1-hydroxybutyl)phényl)méthanesulfonamide ;
le N-(4-(4-(éthyl(6-acétoxy-6-méthylheptyl)amino)-1-hydroxybutyl)phényl)méthanesulfonamide ;
le N-(4-(4-(éthyl(6-hydroxy-6-méthylheptyl)amino)-1-hydroxybutyl)phényl)méthanesulfonamide ; ou
le N-(4-(4-(éthyl(6-fluoro-6-méthylheptyl)amino)-1-hydroxybutyl)phényl)méthanesulfonamide.

## Revendications (Revendications pour l'(les) Etat(s) contractant(s) suivant(s): ES)

1. Procédé de production d'un composé de formule I 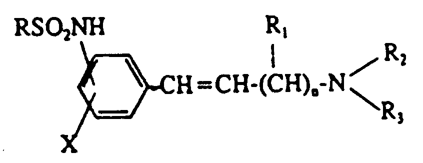 ou d'un sel pharmacologiquement acceptable de ce composé, formule dans laquelle :
n a une valeur de 1 à 3 ;
X représente l'hydrogène, un groupe hydroxy, alkoxy en C₁ à C₄, alkyle en C₁ à C₄, CF₃ ou un halogène ;
R est un groupe alkyle en C₁ à C₄ ;
R₁ est de l'hydrogène ou un groupe alkyle en C₁ à C₄ ; et
R₂ est un groupe alkyle en C₁ à C₁₀ et R₃ représente
a) un groupe alkyle en C₁ à C₁₀,
b) un groupe alkyle en C₁ à C₇ substitué par un radical aryle, hétéroaryle ou cycloalkyle en C₃ à C₇,
c) un groupe alkyle en C₁ à C₁₀ substitué par 1 à 8 atomes de fluor,
d) un groupe cycloalkyle en C₃ à C₁₀,
e) un groupe alcényle en C₃ à C₁₀, ou
f) un groupe alkyle en C₁ à C₁₀ substitué par 1 à 3 substituants hydroxy, acyloxy en C₁ à C₄ ou alkoxy en C₁ à C₅,
sous réserve que la somme des atomes de carbone dans R₂ et R₃ soit supérieure à 5 ; ou bien
NR₂R₃ est un groupe hétérocyclique saturé ayant un atome d'azote et 4 à 8 atomes de carbone ou est une pipérazine substituée en position 4 dans laquelle le substituant en position 4 est un groupe alkyle en C₁ à C₁₀, aryle, benzyle ou hétéroaryle,
qui comprend la déshydratation d'un alcool benzylique correspondant.

2. Procédé suivant la revendication 1, dans lequel X est de l'hydrogène.

3. Procédé suivant la revendication 1 ou la revendication 2, dans lequel R₁ est de l'hydrogène.

4. Procédé suivant l'une quelconque des revendications précédentes, dans lequel R₁ n'apparaît qu'une fois sous forme d'un groupe alkyle.

5. Procédé suivant l'une quelconque des revendications précédentes, dans lequel R₃ est un groupe alkyle à C₁ à C₁₀ substitué par 1 à 8 atomes de fluor.

6. Procédé suivant la revendication 1, dans lequel le composé de formule I est :
le (E)-N-(4-(4-(éthylheptylamino)-1-butényl)phényl)méthanesulfonamide ;
le (E)-N-(4-(4-(hexahydro-1H-azépine-1-yl)-1-butényl)phényl)méthanesulfonamide ;
le (E)-N-(4-(4-(dibutylamino)-1-butényl)phényl)méthanesulfonamide ;
le (E)-N-(4-(4-(dipropylamino)-1-butényl)phényl)isopropanesulfonamide ;
le (E)-N-(4-(4-(heptaméthylène-imino)-1-butényl)phényl)méthanesulfonamide ;
le (E)-N-(3-(4-(éthylheptylamino)-1-butényl)phényl)méthanesulfonamide ;
le (E)-N-(4-(4-(décyléthylamino)-1-butényl)phényl)méthanesulfonamide ;
le (E)-N-(4-(4-(éthyl(6-hydroxyheptyl)amino)-1-butényl)phényl)méthanesulfonamide ;
le (E)-N-(4-(4-(éthyl(2-cyclohexyléthyl)amino)-1-butényl)phényl)méthanesulfonamide ;
le (E)-N-(4-(4-(éthyl(2-cyclopentyléthyl)amino)-1-butényl)phényl)méthanesulfonamide ;
le (E)-N-(4-(4-(éthyl(4,4-diméthylpentyl)amino)-1-butényl)phényl)méthanesulfonamide ;
le (E)-N-(4-(4-éthyl(6-acétoxy-6-méthylheptyl)amino)-1-butényl)phényl)méthanesulfonamide ; ou
le (E)-N-(4-(4-(éthyl(6-fluoro-6-méthylheptyl)amino)-1-butényl)phényl)méthanesulfonamide.

7. Procédé suivant la revendication 1, dans lequel le composé de formule I est :
le(E)-N-(4-(3-(éthylheptylamino)-1-propényl)phényl)méthanesulfonamide ;
le (E)-N-(4-(3-(hexaméthylène-imino)-1-propényl)phényl)méthanesulfonamide ;
le (E)-N-(4-(3-dibutylamino)-1-propényl)phényl)méthanesulfonamide ; ou
le (E)-N-(4-(3-(4-(4-pyridinyl)-1-pipérazinyl)-1-propényl)phényl)méthanesulfonamide.

8. Procédé suivant la revendication 1, dans lequel le composé de formule I est
le (E)-N-(4-hexaméthylène-imino)-1-pentényl)phényl)méthanesulfonamide ; ou
le (E)-N-(4-(dibutylamino)-1-pentényl)phényl)méthanesulfonamide.

9. Procédé suivant l'une quelconque des revendications précédentes, dans lequel la déshydratation est conduite par réaction avec l'acide trifluoracétique dans un solvant à 0-40°C.

10. Utilisation d'un composé de formule I tel que défini dans l'une quelconque des revendications 1 à 8 pour la préparation d'un médicament destiné au traitement de l'arythmie.
